# EUROPEAN PATENT APPLICATION

(11) **EP 0 745 362 A1**
(43) Date of publication of application: **04.12.1996**
(21) Application number: 96830301.6
(22) Date of filing: 24.05.1996
(51) Int. Cl.: A61F 6/00, A61F 6/04

(54) **Condom consisting of two layers joined at the base**

(30) Priority: 02.06.1995 IT RM950129 U
(71) Applicant: Vittorio, Hassan, Roma (IT)
(72) Inventor: Vittorio, Hassan, Roma (IT)
(74) Representative: Baldi, Claudio

(57) **Abstract**

The condom according to this invention consists of two layers (1', 1" ) whose individual thickness is no greater than that of a conventional condom.

Said layers (1', 1") are joined only at the base (2) of the condom.

## Description

This invention concerns a condom that more effectively prevents infection with respect to conventional condoms in those cases when one of the partners has an infectious disease.

It is common knowledge that some diseases such as hepatitis, aids, and to a lesser extent syphilis can be transmitted sexually.

Today, condoms are a fairly secure form of prevention.

The purpose of this invention is to perfect conventional condoms to further reduce the risk of injection. This scope is attained as described in claim The enclosed drawings refer to the following:

Fig. 1 is a conventional single-layer condom.

Fig. 2 is the condom according to this invention.

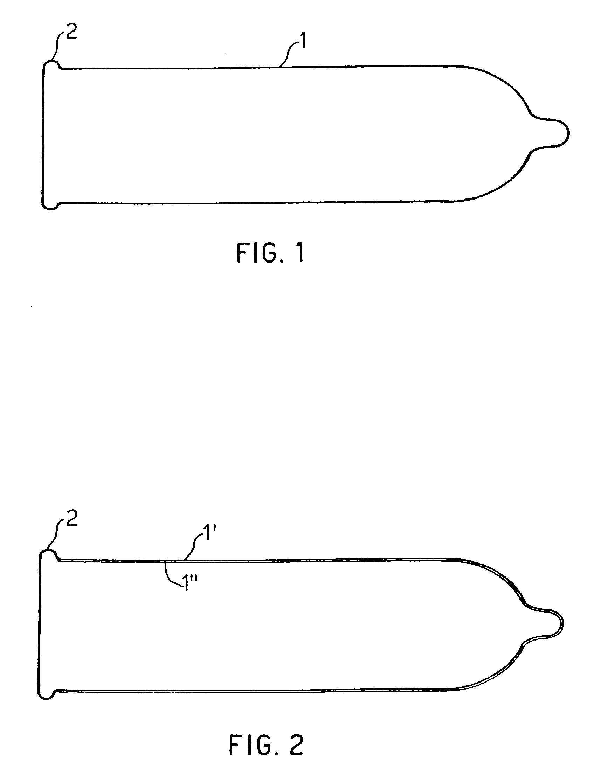

The condom shown in Fig. 2 consists of two layers, more specifically it consists of two conventional condoms 1', 1" (Fig. 1) one of which is fitted in the other and joined at the annular reinforcement section. This joint may be realised by means of any conventional method.

The two layers 1', 1" slide over each other.

During production of the condom, a disinfectant may be inserted and enclosed in the space between the two layers 1', 1".

If external layer 1' breaks, the protection offered by inner layer 1" remains.

Thanks to this double protection of the two layers 1', 1", the thickness of the two layers 1', 1" may be thinner than a normal thickness.

The double condom according to this invention is in addition vulcanised at the base 2.

## Claims

1. A condom characterised by two layers (1', 1") having a thickness d ≤ where D is the thickness of a conventional condom (1), said layers (1', 1") being joined only at the base (2) of the condom and sliding over each other and forming an empty space between the same.

2. A condom according to claim 1, characterised by the fact that the empty space between the two layers (1', 1") is filled with a disinfectant.

3. A condom according to claims 1 and 2 characterised in that the same is vulcanised at base (2).
